# EUROPEAN PATENT APPLICATION

(11) **EP 4 393 398 A1**
(43) Date of publication of application: **03.07.2024**
(21) Application number: 22861441.8
(22) Date of filing: 25.08.2022
(51) Int. Cl.: A61B 5/16

(54) **INFORMATION PROCESSING METHOD, INFORMATION PROCESSING DEVICE, AND INFORMATION PROCESSING PROGRAM**

(30) Priority: 27.08.2021 US 202163237849 P
(71) Applicant: Sony Group Corporation, Tokyo 108-0075 (JP)
(72) Inventor: NIIJIMA, Makoto, Tokyo 108-0075 (JP); AOSHIMA, Chihiro, Tokyo 108-0075 (JP); MISHIMA, Akimasa, Tokyo 108-0075 (JP); ITO, Keisuke, Tokyo 108-0075 (JP); NAKAYAMA, Ryosuke, Tokyo 108-0075 (JP)
(74) Representative: MFG Patentanwälte Meyer-Wildhagen Meggle-Freund Gerhard PartG mbB
(86) International application number: PCT/JP2022/032082
(87) International publication number: WO 2023/027153

(57) **Abstract**

An information processing method according to an embodiment includes the steps of: by a computer, estimating stress information regarding a type of stress of a user on the basis of biological information of the user measured in real time by a biological sensor; and specifying correlation information correlated with the stress information from behavior information related to a behavior history of the user and activity information related to a social activity history of the user depending on the stress information.

## Description

### Field

The present invention relates to an information processing method, an information processing device, and an information processing program.

### Background

Conventionally, for example, there is a serious social problem of mental health disorder in the Japanese society. Therefore, in recent years, technology related to mental health care has been known. For example, questions or measurements are made to a subject, an actual situation model such as experiences of the subject from the past to the present is constructed from answers or results thereof, and trouble of a subject and the cause of the trouble are specified on the basis of the actual situation model that has been constructed. In addition, there is known technology for specifying advice package information for correcting cognitive distortion and resolving the trouble or improving a degree of happiness on the basis of the specified cause of the subject's trouble and the constructed actual situation model, the advice package information to be presented to the subject.

### Citation List

### Patent Literature

Patent Literature 1: JP 2022-59547 A

### Summary

### Technical Problem

However, in the above-described conventional technology, the trouble of the subject and the cause of the trouble are identified merely on the basis of the answers to the questions to the subject or the measurement results, and thus, it is not always possible to improve the usability of the user who uses service related to mental health care.

Therefore, the present disclosure proposes an information processing method, an information processing device, and an information processing program capable of improving usability of a user who uses service related to mental health care.

### Solution to Problem

To solve the above problem, an information processing method according to an embodiment includes the steps of: by a computer, estimating stress information regarding a type of stress of a user on the basis of biological information of the user measured in real time by a biological sensor; and specifying correlation information correlated with the stress information from behavior information related to a behavior history of the user and activity information related to a social activity history of the user depending on the stress information.

### Brief Description of Drawings

FIG. 1 is a diagram illustrating a configuration example of an information processing system according to an embodiment of the present disclosure.
FIG. 2 is a diagram illustrating an example of a sensor device according to the embodiment.
FIG. 3 is a diagram illustrating a configuration example of an information processing device according to the embodiment.
FIG. 4 is a diagram for explaining an example of information processing according to the embodiment.
FIG. 5 is a flowchart illustrating an information processing procedure according to the embodiment.
FIG. 6 is a hardware configuration diagram illustrating an example of a computer that implements functions of the information processing device.

### Description of Embodiments

Hereinafter, embodiments of the present disclosure will be described in detail on the basis of the drawings. Note that in each of the following embodiments, the same parts are denoted by the same symbols, and redundant description will be omitted.

### (Embodiments)

### [1. Introduction]

Conventionally, for example, there is a serious social problem of mental health disorder in the Japanese society. For example, according to a survey by the Ministry of Health, Labour and Welfare (2021), the number one cause of death of young people from 15 to 39 years old living in Japan was suicide. Furthermore, in recent years, the number of suicides has been on an increasing trend in Japan, and the number of suicides in 2021 was 20,000 or more this year. In addition, according to a survey (2020) by the Ministry of Education, Culture, Sports, Science and Technology, the number of students who refuse to go to primary or junior high schools in Japan has increased continuously for 8 years and reached about 200,000. In addition, problems such as suicide and school refusal are considered to be caused by mental health disorder. Therefore, in the Japanese society, there is an increasing need for service related to mental health care.

In addition, conventionally, therapy is known as service related to mental health care. However, there are various problems in the therapy by a human therapist. For example, since the number of therapists is limited, a person in need of therapy (hereinafter, also referred to as a patient) may not receive as much therapy as necessary at any time. For example, since working hours of a therapist are limited, even in a case where there is a person who urgently needs therapy, there are cases where the person cannot receive therapy at night or on holidays. In addition, since the time for one time of therapy is limited, there are cases where sufficient therapy cannot be received in one time of therapy. In addition, there are cases where the person has to go to a place where a therapist is present in order to receive therapy.

In addition, it takes time for the therapist to collect and organize necessary information from the patient. It may also be a burden for the patient to recall his or her memories and to talk in order to provide information to the therapist. In addition, some patients refuse to receive therapy until the physical condition is considerably aggravated, or some patients are not willing to receive therapy that requires meeting a person in the first place. In addition, even in a case where the patient can receive the therapy, there may be a problem such as poor compatibility with the therapist. Furthermore, for example, in a case where a personal relationship at home, a school, a workplace, or the like is the cause of the stress for the patient, it may be difficult to grasp the actual state only on the basis of information obtained from a dialogue with the patient.

Therefore, an information processing device 100 according to an embodiment of the present disclosure estimates stress information regarding the type of stress of a user on the basis of the biological information of the user measured in real time by a biological sensor. Furthermore, the information processing device 100 specifies correlation information correlated with the stress information from behavior information regarding a behavior history of the user and activity information regarding the social activity history of the user depending on the stress information.

As a result, the information processing device 100 can quickly estimate the type of stress of the user on the basis of the biological information of the user measured in real time by the biological sensor. Furthermore, since the information processing device 100 can quickly detect aggravation of the physical condition of the user, it is possible to quickly determine whether or not therapy for the user is necessary. Furthermore, in a case where it is determined that the therapy for the user is necessary, the information processing device 100 can quickly execute a program for executing the therapy instead of a human therapist. As a result, the information processing device 100 can quickly provide therapy to a user in need of therapy anytime (for example, even at night or on holidays) anywhere (for example, at home or other places). In addition, since the information processing device 100 can quickly provide therapy to a user in need of therapy anytime and anywhere, it is possible to provide care to the mental health while the user's mental health disorder is still mild. Therefore, the information processing device 100 can prevent, in advance, aggravation of the mental health disorder of the user who needs the therapy. Furthermore, the information processing device 100 can prevent, in advance, aggravation of the mental health disorder of the user in need of the therapy and escalation to suicide or school refusal. Furthermore, the information processing device 100 can provide therapy to the user in need of therapy with extra time.

Furthermore, the information processing device 100 makes it possible to appropriately specify the cause of stress on the basis of the correlation information correlated with the stress information, in addition to the information obtained through dialogue with the user, by specifying the correlation information correlated with the stress information from among the behavior information regarding the behavior history of the user and the activity information regarding the social activity history of the user depending on the stress information. Furthermore, since the information processing device 100 can appropriately identify the cause of stress, appropriate therapy can be provided. The information processing device 100 can also reduce the burden of information provision of the user who receives the therapy.

### [2. Configuration of Information Processing System]

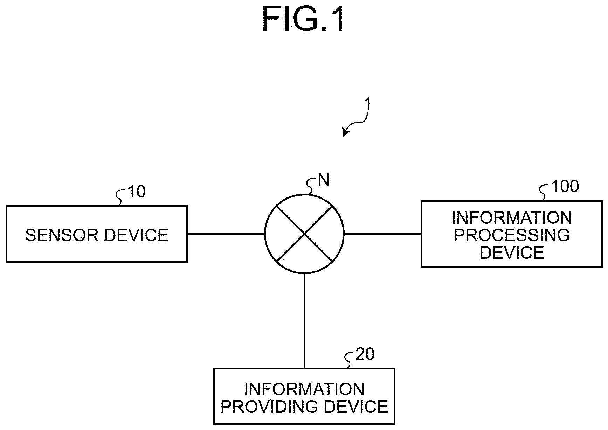

FIG. 1 is a diagram illustrating a configuration example of an information processing system 1 according to an embodiment of the present disclosure. The information processing system 1 includes a sensor device 10, an information providing device 20, and the information processing device 100. The sensor device 10, the information providing device 20, and the information processing device 100 are communicably connected to each other in a wired or wireless manner via a predetermined network N. Note that the information processing system 1 illustrated in FIG. 1 may include any number of sensor devices 10, any number of information providing devices 20, and any number of information processing devices 100.

The sensor device 10 is an information processing device that includes a biological sensor and measures biological information of the user in real time by the biological sensor. Specifically, the sensor device 10 is always worn by the user and constantly monitors biological information of the user. More specifically, the sensor device 10 measures, as the biological information of the user, the amount of hormones in the blood of the user, the blood pressure, the heart rate, the blood glucose level, or a measurement value regarding the electroencephalography of the user in real time. For example, the sensor device 10 measures measurement values regarding the amount of cortisol, dopamine, adrenaline, noradrenaline, oxytocin, endorphin, or serotonin as the hormones in the blood of the user in real time. The sensor device 10 measures the biological information of the user in real time by the biological sensor and transmits the biological information of the user measured in real time by the biological sensor to the information processing device 100.

Here, the roles of various hormones in blood will be described. Cortisol, also called the "stress hormone", is a hormone whose secretion from the adrenal gland increases when the body and the mind are stressed. Dopamine, also called the "reward hormone", is a hormone that emotionally excites or makes a person on tension. Adrenaline, also called the "fight or flight hormone", is a hormone having a strong physical action such as blood vessels or muscles. Noradrenaline, also called the "irritation hormone", is a hormone that has strong mental effects such as surprise, excitement, or fear. Oxytocin is also called the "love hormone". Endorphin, also called the "intracerebral narcotics", is a hormone that gives a strong uplifting feeling. Serotonin, also referred to as the "happy hormone", is a hormone related to reassurance and relaxation.

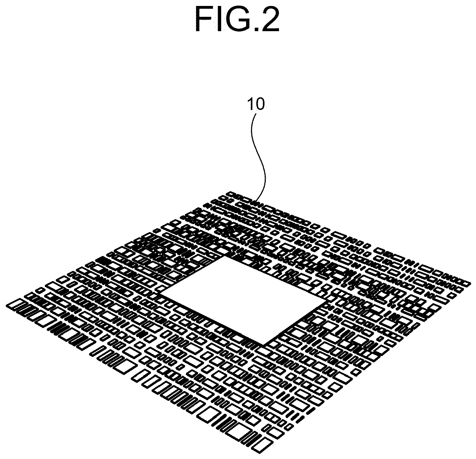

FIG. 2 is a diagram illustrating an example of the sensor device according to the embodiment of the disclosure. The sensor device illustrated in FIG. 2 is a biological sensor that has a thin film shape and can be worn at all times by being attached to the skin. The sensor device illustrated in FIG. 2 is applied with the technology of laser doppler velocimeter (LDV) and measures the flow rate of blood by light. For example, the speed of the blood fluid or the amount of hormones in the blood at a part of the body, to which the sensor device is attached, is measured by utilizing the coherence of laser light emitted from the part to which the sensor device is attached.

The information providing device 20 is a server device that provides behavior information regarding the behavior history of the user and activity information regarding the social activity history of the user. In response to a request from the information processing device 100, the information providing device 20 provides, to the information processing device 100, the behavior information regarding the behavior history of the user and the activity information regarding the social activity history of the user.

The behavior information is also called personal terminal history basic data (or personal information) of the user and includes information regarding various histories acquired from a terminal device of the user. Specifically, the behavior information is position information regarding a position history of the user, search information regarding a search history, browsing information regarding a browsing history, purchase information regarding a purchase history, travel information regarding a travel history, posting information regarding a posting history, or information regarding a history of image data or audio data stored in the terminal device of the user. For example, the behavior information includes information posted on an SNS by the user, a shopping history, a position information history of a trip, a video captured by a camera of the terminal device of the user, voice data recorded using a recording function of the terminal device of the user, or information regarding a history of message exchange by e-mails, LINE (registered trademark), or the like. The information providing device 20 acquires the behavior information of the user from the terminal device of the user. For example, the information providing device 20 may acquire relatively recent behavior information of the user such as behavior information in the last past one month. Note that the information providing device 20 may acquire behavior information in any period such as the latest past three months or the latest past one week without being limited to the behavior information in the latest past one month.

The activity information is also referred to as social security system basic data (or social security basic information). Specifically, the activity information is personal information managed by a country, a national institution, or a national public organization to which the user has ever belonged or an organization to which the user has ever belonged or health information regarding the health of the user. For example, the information providing device 20 may acquire personal information of the user managed by the Ministry of Health, Labour and Welfare. Furthermore, the information providing device 20 may acquire personal information (for example, personal information such as the family structure, a marriage history, a divorce history, and the amount of taxes) of the user managed by a local government where the user lives. In addition, the information providing device 20 may acquire personal information (for example, personal information such as the amount of income, a qualification possessed by the user, a position, a department to which the user belongs, a history of personnel transfer, and personnel evaluation) of the user managed by a personnel department of a company to which the user belongs. Furthermore, the information providing device 20 may acquire personal information (for example, performance in a school, and the like) of the user managed by a school where the user went. Furthermore, the information providing device 20 may acquire health information (for example, a medical record of the user, a result of a medical check-up, and the like) regarding the health of the user from a hospital or the like where the user has ever used.

The information processing device 100 is a computer used by the user. Specifically, the information processing device 100 is a device (terminal device) that can be carried by the user. The information processing device 100 is implemented by a smartphone, a tablet terminal, a laptop personal computer (PC), a mobile phone, a personal digital assistant (PDA), or the like.

Furthermore, the information processing device 100 acquires the biological information of the user measured in real time by the biological sensor from the sensor device 10. Subsequently, the information processing device 100 estimates stress information regarding the type of stress of the user on the basis of the biological information of the user measured in real time by the biological sensor.

The information processing device 100 also acquires the behavior information regarding the behavior history of the user and the activity information regarding the social activity history of the user from the information providing device 20. Subsequently, the information processing device 100 specifies correlation information correlated with the stress information from the behavior information regarding the behavior history of the user and the activity information regarding the social activity history of the user depending on the stress information that has been estimated.

Subsequently, the information processing device 100 gives therapy to the user on the basis of the specified correlation information and specifies the cause of the stress of the user. In addition, the information processing device 100 executes a recovery program corresponding to the cause of the stress of the user.

### [3. Configuration of Information Processing Device]

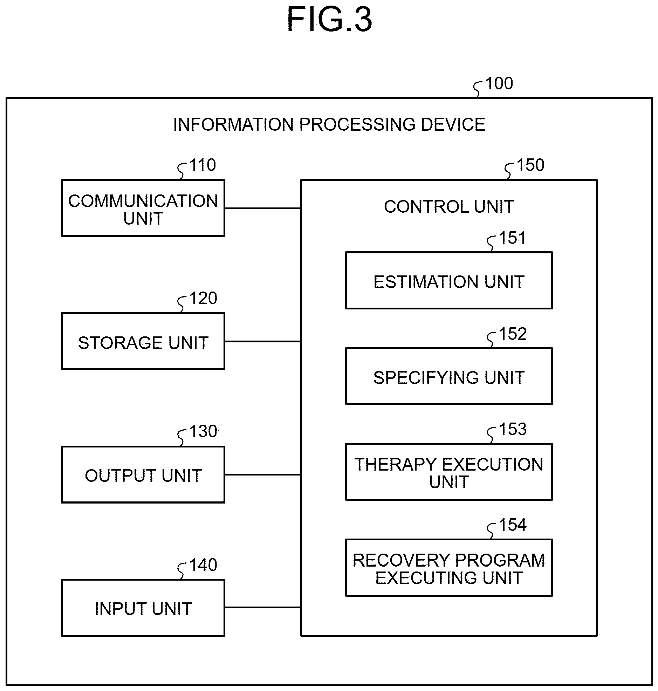

FIG. 3 is a diagram illustrating a configuration example of the information processing device 100 according to the embodiment of the disclosure. As illustrated in FIG. 3, the information processing device 100 includes a communication unit 110, a storage unit 120, an output unit 130, an input unit 140, and a control unit 150.

The communication unit 110 is implemented by, for example, a network interface card (NIC) or the like. The communication unit 110 is connected to the network N in a wired or wireless manner and, for example, transmits and receives information to and from the sensor device 10 or the information providing device 20.

The storage unit 120 is implemented by, for example, a semiconductor memory element such as a random access memory (RAM) or a flash memory or a storage device such as a hard disk or an optical disk. For example, the storage unit 120 stores information regarding a first machine learning model and a second machine learning model to be described later. Furthermore, the storage unit 120 stores information regarding a recovery program to be described later.

The output unit 130 outputs various types of information. The output unit 130 includes, for example, a liquid crystal display, an organic electro-luminescence (EL) display, or the like and displays various types of information. Furthermore, the output unit 130 includes, for example, a speaker, and outputs various types of information by audio. Note that the audio output may be performed not by the output unit 130 but by an external speaker, a headphone, or others connected via the communication unit 110.

The input unit 140 receives various input operations from the user. The input unit 140 includes, for example, a microphone, and receives audio input from the user. Furthermore, the input unit 140 includes, for example, a keyboard, a mouse, or a touch panel in a case of a smartphone and receives various input operations from the user.

The control unit 150 is a controller and is implemented by, for example, a central processing unit (CPU), a micro processing unit (MPU), an application specific integrated circuit (ASIC), a field programmable gate array (FPGA), or the like executing various programs (corresponding to an example of an information processing program) stored in a storage device inside the information processing device 100 using a storage area such as a RAM as a work area. In the example illustrated in FIG. 2, the control unit 150 includes an estimation unit 151, a specifying unit 152, a therapy execution unit 153, and a recovery program executing unit 154.

The estimation unit 151 acquires the biological information of the user measured in real time by the biological sensor from the sensor device 10. Specifically, the estimation unit 151 acquires measurement values related to the amount of hormones in the blood of the user as the biological information. For example, the estimation unit 151 acquires measurement values regarding the amount of cortisol, dopamine, adrenaline, noradrenaline, oxytocin, endorphin, or serotonin as the hormones in the blood of the user. Furthermore, the estimation unit 151 acquires measurement values regarding the blood pressure, the heart rate, the blood glucose level, or the electroencephalography of the user as the biological information.

Subsequently, the estimation unit 151 estimates stress information regarding the type of stress of the user on the basis of the biological information of the user measured in real time by the biological sensor. Specifically, in a case where measurement values related to a plurality of types of biological information indicate abnormal values, the estimation unit 151 estimates stress information on the basis of the pattern of the combination of the abnormal values indicated by the measurement values related to the plurality of types of biological information. Here, a measurement value related to the biological information indicating an abnormal value means that the measurement value related to the biological information has a value outside the range of reference values related to the biological information. More specifically, in a case where the measurement values related to the plurality of types of biological information of the user are input to the first machine learning model as the input information, the estimation unit 151 estimates the stress information of the user using the first machine learning model learned in advance so as to output, as output information, probabilities of falling into each combination pattern of abnormal values indicated by the measurement values related to the plurality of types of biological information.

For example, a first combination pattern of abnormal values indicated by measurement values related to a plurality of types of biological information corresponds to a first stress type (for example, stress from growing up in a dysfunctional family). Meanwhile, a second combination pattern of abnormal values indicated by the measurement values related to a plurality of types of biological information corresponds to a second stress type (for example, stress due to uneasiness about health). In addition, a third combination pattern of abnormal values indicated by the measurement values related to a plurality of types of biological information corresponds to a third stress type (for example, stress due to responsibility at work). At this point, in the case where the measurement values related to the plurality of types of biological information of the user are input to the first machine learning model as the input information, and in a case where the measurement values related to the plurality of types of biological information do not indicate any abnormal values, the estimation unit 151 calculates probabilities that the measurement values related to the plurality of types of biological information correspond to the first, second, and third combination patterns of abnormal values as zero for each of them and outputs the probabilities as output information. Meanwhile, in the case where the measurement values related to the plurality of types of biological information of the user are input to the first machine learning model as the input information, and in a case where the measurement values related to the plurality of types of biological information indicate any abnormal values, the estimation unit 151 calculates probabilities that the measurement values related to the plurality of types of biological information correspond to the first, second, and third combination patterns of abnormal values and outputs the probabilities as output information of the first machine learning model. For example, the estimation unit 151 calculates probabilities of corresponding to the first, second, and third combination patterns of abnormal values as 80%, 10%, and 10%, respectively, and outputs the probabilities as output information of the first machine learning model. Subsequently, the estimation unit 151 estimates that the type of stress, corresponding to the pattern of the combination of abnormal values corresponding to the highest probability among the probabilities output as the output information of the first machine learning model, is the stress information of the user.

Furthermore, the estimation unit 151 may estimate the stress information on the basis of a transition pattern of measurement values related to the biological information in addition to the combination pattern of abnormal values indicated by the measurement values regarding the plurality of types of biological information. At this point, a transition pattern of a measurement value regarding the biological information can be rephrased as a pattern of temporal changes of the measurement value regarding the biological information or a pattern of chronological changes of the measurement value regarding the biological information. More specifically, in a case where transition patterns of the measurement values related to the biological information of the user are input to the first machine learning model as input information, the estimation unit 151 estimates the stress information of the user using the first machine learning model learned in advance so as to output, as output information, probabilities of falling into the transition patterns of the measurement values related to the biological information.

For example, a first transition pattern of measurement values related to the biological information corresponds to a first stress type (for example, stress from growing up in a dysfunctional family). Meanwhile, a second transition pattern of the measurement values related to the biological information corresponds to a second stress type (for example, stress due to uneasiness about health). Meanwhile, a third transition pattern of the measurement values related to the biological information corresponds to a third stress type (for example, stress due to responsibility at work). At this point, in a case where the transition pattern of the measurement values regarding the biological information of the user is input to the first machine learning model as input information, in a case where the transition pattern of the measurement values regarding the biological information does not correspond to any of the first, second, and third transition patterns of the measurement values, the estimation unit 151 calculates the probabilities that the transition pattern of the measurement values regarding the biological information corresponds to the first, second, and third transition patterns of the measurement values as zero for each of them and outputs the probabilities as output information. Meanwhile, in a case where the transition pattern of the measurement values regarding the biological information of the user is input to the first machine learning model as input information, in a case where the transition pattern of the measurement values regarding the biological information corresponds to any of the first, second, and third transition patterns of the measurement values, the estimation unit 151 calculates the probabilities that the transition pattern of the measurement values regarding the biological information corresponds to the first, second, and third transition patterns of the measurement values and outputs the probabilities as output information. For example, the estimation unit 151 calculates probabilities of corresponding to the first, second, and third transition patterns of the measurement values as 70%, 20%, and 10%, respectively, and outputs the probabilities as output information of the first machine learning model. Subsequently, the estimation unit 151 estimates that the type of stress, corresponding to the transition pattern of the measurement values corresponding to the highest probability among the probabilities output as the output information of the first machine learning model, is the stress information of the user.

Note that the estimation unit 151 may estimate the stress information in a case where at least one of the measurement values regarding the amounts of cortisol, dopamine, adrenaline, noradrenaline, oxytocin, endorphin, or serotonin or the measurement values regarding the blood pressure, the heart rate, the blood glucose level, or the electroencephalography of the user indicates an abnormal value. For example, the estimation unit 151 may estimate the stress information depending on the type of a hormone indicating an abnormal value. For example, in a case where a measurement value regarding the amount of noradrenaline indicates an abnormal value, the estimation unit 151 may estimate that the user is in a state of stress in which mental processes such as surprise, excitement, or fear are strong. In this manner, the estimation unit 151 may estimate the stress information on the basis of whether or not the measurement values related to the biological information indicate an abnormal value.

The specifying unit 152 specifies correlation information correlated with the stress information from the behavior information regarding the behavior history of the user and the activity information regarding the social activity history of the user depending on the stress information estimated by the estimation unit 151. For example, the specifying unit 152 specifies a keyword corresponding to the stress information estimated by the estimation unit 151 by referring to a first list in which stress information and keywords corresponding to the stress information are associated with each other in advance. Subsequently, the specifying unit 152 searches for correlation information correlated with the stress information from among the behavior information and the activity information of the user on the basis of the specified keyword. For example, in a case where the type of stress of the user estimated by the estimation unit 151 is "stress from growing up in a dysfunctional family", the specifying unit 152 refers to the first list and specifies keywords such as "family", "home", or "relationship" as keywords corresponding to the "stress from growing up in a dysfunctional family". Subsequently, the specifying unit 152 searches for correlation information correlated with the stress information from among the behavior information and the activity information of the user on the basis of the specified keywords such as "family", "home", or "relationship". For example, the specifying unit 152 specifies personal information regarding the family structure as correlation information correlated with the stress information from the activity information of the user. In addition, the specifying unit 152 specifies the personnel information in a company as correlation information correlated with the stress information from the activity information of the user. In addition, the specifying unit 152 refers to a list in which the stress information and the type of behavior information or activity information to be searched for specifying the correlation information corresponding to the stress information are associated with each other in advance and specifies the type of the behavior information or the activity information corresponding to the stress information estimated by the estimation unit 151. Subsequently, the specifying unit 152 searches for correlation information correlated with the stress information from among the behavior information and the activity information of the user on the basis of the specified type of behavior information or activity information. Furthermore, in a case where the type of the stress of the user estimated by the estimation unit 151 is "stress from growing up in a dysfunctional family", the specifying unit 152 refers to a second list and identifies, for example, information regarding posted information of an SNS, a video captured by a terminal device, or audio recorded by the terminal device as the type of behavior information corresponding to the "stress from growing up in a dysfunctional family". Subsequently, the specifying unit 152 specifies information related to the posted information of the SNS, the video captured by the terminal device, and the audio recorded by the terminal device as the correlation information correlated with the stress information from the behavior information of the user.

The therapy execution unit 153 executes therapy for the user on the basis of the correlation information specified by the specifying unit 152. Specifically, the therapy execution unit 153 outputs question information regarding a question for the user, acquires reaction information regarding a reaction of the user to the question, and identifies the cause of the stress of the user on the basis of the reaction information and the correlation information. For example, in a case where the reaction information and the correlation information are input to the second machine learning model as input information, the therapy execution unit 153 specifies the cause of the stress of the user by using the second machine learning model learned in advance so as to output information regarding the cause of the stress of the user as output information. Here, the reaction information may be answer information regarding an answer of the user to the question. Incidentally, the reaction information may be biological information of the user measured during the therapy for the user.

Furthermore, the therapy execution unit 153 may specify a schema of the user on the basis of the reaction information and the correlation information. Incidentally, the schema is a term used in cognitive psychology, which is one of concepts used to describe human cognitive processes. For example, the schema is structured knowledge that supports cognitive activities such as external perception, use of a language, and thinking. That is, a schema is something like a pattern of thought.

The recovery program executing unit 154 executes a recovery program corresponding to the cause of the stress specified by the therapy execution unit 153. Specifically, the recovery program executing unit 154 selects a recovery program corresponding to the cause of the stress. For example, the recovery program executing unit 154 selects a program that enables the user to release stress, such as mindfulness or karaoke, depending on the cause of the stress. In addition, in a case where the schema of the user is specified, the recovery program executing unit 154 selects a recovery program for correcting the cognitive distortion corresponding to the schema of the user. Subsequently, the recovery program executing unit 154 executes the selected recovery program.

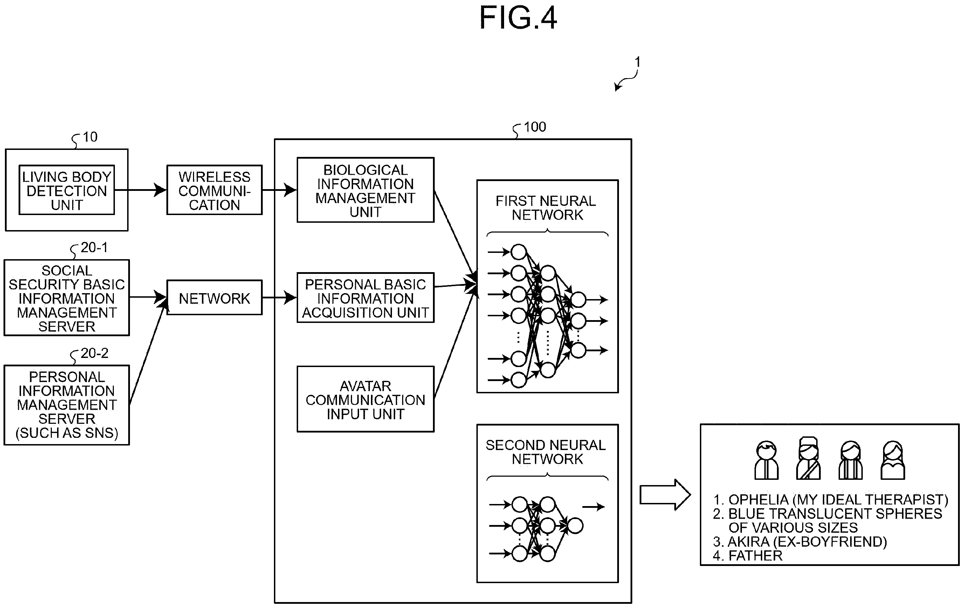

FIG. 4 is a diagram for explaining an example of information processing according to the embodiment of the present disclosure. The sensor device 10 illustrated in FIG. 4 includes a living body detection unit. Since the sensor device 10 is always worn by the user to be detected, the living body detection unit continuously detects a plurality of pieces of biological information of the user on a long-term basis. The living body detection unit continuously transmits the plurality of pieces of biological information of the user to the information processing device 100 by wireless communication on a long-term basis. The living body detection unit continuously transmits, to the information processing device 100, biological information reflecting the influence of activities of the user, such as the diet, exercises, sleep, and others of the user and variations due to time or season on a long-term basis.

Furthermore, the information processing device 100 illustrated in FIG. 4 includes a biological information management unit. The biological information management unit corresponds to the estimation unit 151 described in FIG. 3. The information processing device 100 continuously acquires a plurality of pieces of biological information of the user from the sensor device 10 on a long-term basis. That is, the information processing device 100 continuously acquires biological information reflecting the influence of activities of the user, such as the diet, exercise, sleep, and others and variations due to time or season on a long-term basis. Furthermore, the information processing device 100 causes a first neural network (for example, corresponds to the first machine learning model described above) to learn, as learning data, a plurality of pieces of biological information of the user continuously detected on a long-term basis. As a result, the information processing device 100 can cause the first neural network to learn the range of a reference value related to the biological information of the user.

Furthermore, the information processing device 100 acquires biological information of not only one user but also a large number of users (for example, all users wearing the sensor device 10) and causes the first neural network to learn using the acquired biological information of the large number of users as learning data. As a result, the information processing device 100 can always update what state is generally in the range of the reference value related to the biological information. In addition, the information processing device 100 causes the first neural network to learn what type of stress symptom the combination of the abnormal values of the biological information is strongly correlated with. In addition, the information processing device 100 accumulates classifications considered to be typical examples of stress symptoms as various types of stress indicators and causes the first neural network to learn. In a case where it is determined that the measurement values regarding the biological information of the user indicate an abnormal value, the information processing device 100 uses the first neural network to determine which stress indicator the stress symptom of the user is close to.

Furthermore, the information processing device 100 illustrated in FIG. 4 includes a personal basic information acquisition unit. The personal basic information acquisition unit corresponds to the estimation unit 151 described in FIG. 3. In a case where the information processing device 100 determines which stress indicator the stress symptom of the user is close to, the information processing device 100 acquires the social security basic information of the user from a social security basic information management server 20-1. Incidentally, the social security basic information (performance or records in a school, personnel information in a company, medical data or medical check-up data, and the like) is personal information managed by a community to which a patient belongs, such as a country, a company, or a school. In the present embodiment, it is based on the premise that a community to which a patient belongs, such as a country, a company, or a school, has agreed to regularly use these pieces of data. Furthermore, the information processing device 100 can access these information records as one of the causes of stress. For example, the information processing device 100 causes the first neural network to learn papers or diagnostic data regarding the causal relationship that information or a record indicating a relatively abnormal experience may have been a cause of a stress symptom. For example, the information processing device 100 may specify information or a record indicating a relatively abnormal experience in the social security basic information as correlation information correlated with the stress information by using the first neural network.

Furthermore, in a case where the information processing device 100 determines which stress indicator the stress symptom of the user is close to, the information processing device 100 acquires the personal behavior history of the user from a personal information management server (such as an SNS server) 20-2. Incidentally, the personal behavior history (search history or comments on an SNS, shopping or trip history, video or recording, etc.) is data owned by an individual by a smartphone, a money payment system, recording of a camera or a microphone, or the like. In the present embodiment, since these pieces of data are backed up in the servers, it is based on the premise that the user agrees to regularly use the data. The information processing device 100 can also access these information records as one of the causes of stress. The information processing device 100 specifies correlation information correlated with the stress information from the personal behavior history.

Subsequently, in a case where the correlation information is input to the first neural network (for example, corresponding to the above-described second machine learning model) as input information, the information processing device 100 specifies the cause of the stress of the user by using the first neural network model learned in advance so as to output the information regarding the cause of the stress of the user as output information. The above is what the information processing device 100 automatically performs, after determining which stress indicator the stress symptom of the user is close to, in order to determine the cause of the stress of the user.

Furthermore, in a case where the cause of the stress of the user has been specified to a certain extent, the information processing device 100 displays an avatar of a therapist (hereinafter, the avatar) on a screen and clearly notifies the user that "the healthcare program has been activated". In the example illustrated in FIG. 4, the information processing device 100 causes the second neural network to learn in advance so as to select and display an avatar that matches the stress symptom or the cause of the stress of the user from among avatars (planar image or three-dimensional image) each corresponding to an ideal therapist of the user, blue translucent spheres of various sizes, an ex of the user, or the father of the user. The information processing device 100 uses the second neural network to display, on the screen, the avatar corresponding to the stress symptom or the cause of the stress of the user. Furthermore, the information processing device 100 appropriately controls a transmission means (for example, tone of voice, wording, and others) from the avatar to the user depending on the avatar.

From this point, the information processing device 100 starts a conversation in a natural language, namely, an interview, by outputting audio. Note that, in a case where it is determined that the user has hearing impairment, the information processing device 100 may conduct an interview by displaying a text. The information processing device 100 collects information of facts by asking a question to the user and encouraging the user to talk about the fact or feelings and uses the information for selecting a recovery program. Furthermore, also at this point, the sensor device 10 measures reaction information (biological information) of the user to the question. Furthermore, the information processing device 100 acquires the reaction information from the sensor device 10. Subsequently, in a case where the reaction information is input to the first neural network (for example, corresponding to the above-described second machine learning model) as input information, the information processing device 100 specifies the cause of the stress of the user by using the first neural network model learned in advance so as to output the information regarding the cause of the stress of the user as output information. Furthermore, the information processing device 100 uses the first neural network model learned in advance so as to determine an unconscious reaction (lie or oscillation) on the basis of the biological information to read the unconscious reaction (lie or restlessness) of the user from the reaction information (biological information) of the user to the question. As a result, the information processing device 100 can specify the cause of the stress of the user further scientifically.

### [4. Information Processing Procedure]

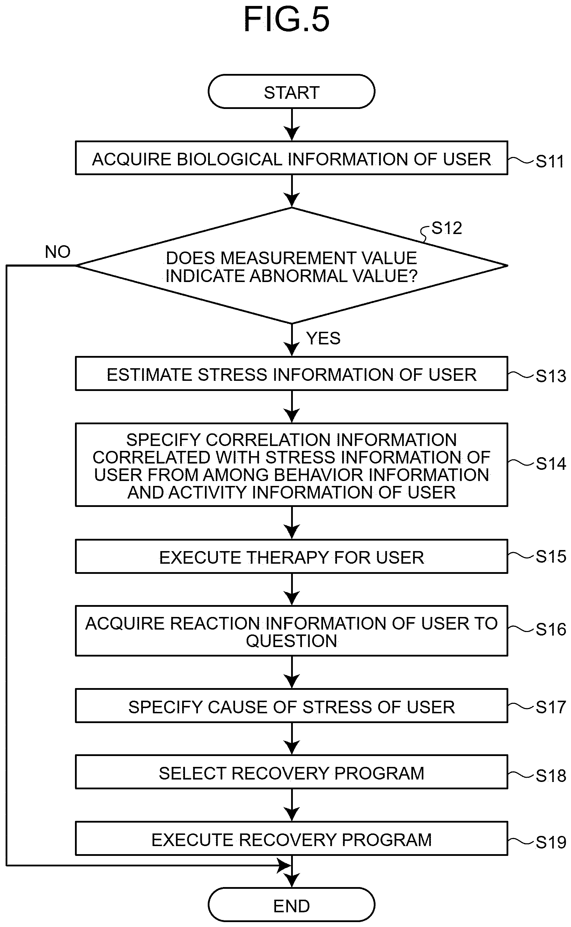

FIG. 5 is a flowchart illustrating the procedure of the information processing according to the embodiment of the present disclosure. As illustrated in FIG. 5, the estimation unit 151 acquires the biological information of the user measured in real time by the biological sensor (Step S11). Subsequently, the estimation unit 151 determines whether or not a measurement value related to the biological information of the user indicates an abnormal value (Step S12). If the estimation unit 151 determines that the measurement value related to the biological information of the user does not indicate an abnormal value (Step S12; No), the processing ends. On the other hand, if the estimation unit 151 determines that the measurement value related to the biological information of the user indicates an abnormal value (Step S12; Yes), stress information of the user is estimated (Step S13).

In addition, the specifying unit 152 specifies correlation information correlated with the stress information of the user from among the behavior information and the activity information of the user depending on the stress information of the user estimated by the estimation unit 151 (Step S14).

Furthermore, the therapy execution unit 153 executes therapy for the user on the basis of the correlation information specified by the specifying unit 152 (Step S15). Subsequently, the therapy execution unit 153 acquires reaction information of the user to the question (Step S16). Then, the therapy execution unit 153 specifies the cause of the stress of the user on the basis of the reaction information and the correlation information (Step S17).

In addition, the recovery program executing unit 154 selects a recovery program corresponding to the cause of stress specified by the therapy execution unit 153 (Step S18). Then, the recovery program executing unit 154 executes the selected recovery program (Step S19).

### [5. Effects]

As described above, the information processing device 100 according to the embodiment or the modification of the present disclosure includes the estimation unit 151 and the specifying unit 152. The estimation unit 151 estimates stress information regarding the type of stress of the user on the basis of the biological information of the user measured in real time by the biological sensor. The specifying unit 152 specifies correlation information correlated with the stress information from the behavior information regarding the behavior history of the user and the activity information regarding the social activity history of the user depending on the stress information.

As a result, the information processing device 100 can quickly estimate the type of stress of the user on the basis of the biological information of the user measured in real time by the biological sensor. Furthermore, since the information processing device 100 can quickly detect aggravation of the physical condition of the user, it is possible to quickly determine whether or not therapy for the user is necessary. Therefore, the information processing device 100 can prevent, in advance, aggravation of the mental health disorder of the user who needs the therapy. Furthermore, the information processing device 100 can prevent, in advance, aggravation of the mental health disorder of the user in need of the therapy and escalation to suicide or school refusal. Furthermore, the information processing device 100 can provide therapy to the user in need of therapy with extra time. Furthermore, the information processing device 100 makes it possible to appropriately specify the cause of stress on the basis of the correlation information correlated with the stress information, in addition to the information obtained through dialogue with the user, by specifying the correlation information correlated with the stress information from among the behavior information regarding the behavior history of the user and the activity information regarding the social activity history of the user depending on the stress information. Furthermore, since the information processing device 100 can appropriately identify the cause of stress, appropriate therapy can be provided. The information processing device 100 can also reduce the burden of information provision of the user who receives the therapy.

Furthermore, the estimation unit 151 estimates the stress information on the basis of whether or not the measurement values related to the biological information indicate an abnormal value.

As a result, the information processing device 100 can appropriately estimate the type of the stress of the user on the basis of whether or not the measurement values related to the biological information indicate an abnormal value.

Furthermore, in a case where measurement values related to a plurality of types of biological information indicate abnormal values, the estimation unit 151 estimates stress information on the basis of the pattern of the combination of the abnormal values indicated by the measurement values related to the plurality of types of biological information.

As a result, the information processing device 100 can appropriately estimate the type of the stress of the user on the basis of the pattern of the combination of the abnormal values indicated by the measurement values related to the plurality of types of biological information.

Furthermore, the estimation unit 151 estimates the stress information on the basis of the transition pattern of the measurement values regarding the biological information.

As a result, the information processing device 100 can appropriately estimate the type of the stress of the user on the basis of the transition pattern of the measurement values related to the biological information.

Incidentally, the biological information is the amount of hormones in the blood of the user.

As a result, the information processing device 100 can appropriately estimate the type of the stress of the user on the basis of the measurement values related to the amount of hormones in the blood of the user.

Incidentally, the biological information is the blood pressure, the heart rate, the blood glucose level, or the electroencephalography of the user.

As a result, the information processing device 100 can appropriately estimate the type of the stress of the user on the basis of the measurement values related to the blood pressure, the heart rate, the blood glucose level, or the electroencephalography of the user.

In addition, the behavior information is position information regarding a position history of the user, search information regarding a search history, browsing information regarding a browsing history, purchase information regarding a purchase history, travel information regarding a travel history, posting information regarding a posting history, or information regarding a history of image data or audio data stored in the terminal device of the user.

As a result, the information processing device 100 can specify the correlation information correlated with the stress information of the user from the relatively recent behavior information of the user such as the behavior information in the last past one month. Therefore, the information processing device 100 can appropriately specify the cause of the stress of the user on the basis of the relatively recent behavior information of the user.

In addition, the activity information is personal information managed by a country, a national institution, or a national public organization to which the user has ever belonged or an organization to which the user has ever belonged or health information regarding the health of the user.

As a result, the information processing device 100 can specify correlation information correlated with the stress information of the user from the past activity information of the user such as activity information of ten or more years ago. Therefore, the information processing device 100 can appropriately specify the cause of the stress of the user on the basis of the past activity information of the user.

Furthermore, the information processing device 100 further includes the therapy execution unit 153. The therapy execution unit 153 executes the therapy for the user on the basis of the correlation information. Specifically, the therapy execution unit 153 outputs question information regarding a question for the user, acquires reaction information regarding a reaction of the user to the question, and identifies the cause of the stress of the user on the basis of the reaction information and the correlation information.

As a result, in a case where it is determined that the therapy for the user is necessary, the information processing device 100 can quickly execute a program for executing the therapy instead of a human therapist. As a result, the information processing device 100 can quickly provide therapy to a user in need of therapy anytime (for example, even at night or on holidays) anywhere (for example, at home or other places). In addition, since the information processing device 100 can quickly provide therapy to a user in need of therapy anytime and anywhere, it is possible to provide care to the mental health while the user's mental health disorder is still mild.

Furthermore, the therapy execution unit 153 specifies the schema of the user on the basis of the reaction information and the correlation information.

As a result, the information processing device 100 can appropriately specify the schema of the user on the basis of the reaction information and the correlation information.

Incidentally, the reaction information is answer information regarding the answer of the user to the question.

As a result, the information processing device 100 can appropriately specify the schema of the user on the basis of the answer information and the correlation information.

Incidentally, the reaction information is biological information of the user measured during the therapy for the user.

As a result, the information processing device 100 can appropriately specify the schema of the user on the basis of the biological information and the correlation information of the user measured during the therapy.

Furthermore, the information processing device 100 further includes the recovery program executing unit 154. The recovery program executing unit 154 executes a recovery program corresponding to the cause of the stress.

As a result, the information processing device 100 can prompt the user to execute the recovery program corresponding to the cause of the stress and thus can support recovery of the user from the mental health disorder.

In addition, in a case where the schema of the user is specified, the recovery program executing unit 154 executes a recovery program for correcting the cognitive distortion corresponding to the schema of the user.

As a result, the information processing device 100 can support recovery from mental health disorder of the user by correcting the cognitive distortion corresponding to the schema of the user.

### [6. Hardware Configuration]

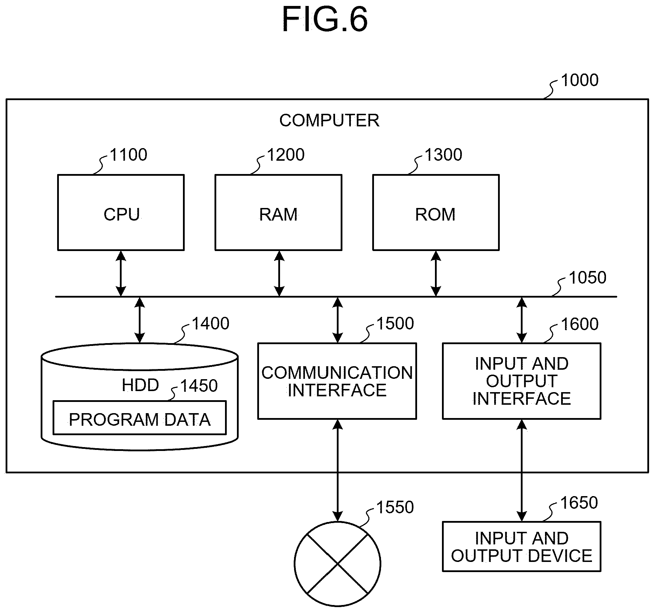

An information device such as the information processing device 100 according to the embodiment described above is implemented by, for example, a computer 1000 having a configuration as illustrated in FIG. 6. FIG. 6 is a hardware configuration diagram illustrating an example of the computer 1000 that implements functions of an information processing device such as the information processing device 100. Hereinafter, the information processing device 100 according to the embodiment will be described as an example. The computer 1000 includes a CPU 1100, a RAM 1200, a read only memory (ROM) 1300, a hard disk drive (HDD) 1400, a communication interface 1500, and an input and output interface 1600. The components of the computer 1000 are connected by a bus 1050.

The CPU 1100 operates in accordance with a program stored in the ROM 1300 or the HDD 1400 and controls each of the components. For example, the CPU 1100 loads a program stored in the ROM 1300 or the HDD 1400 in the RAM 1200 and executes processing corresponding to various programs.

The ROM 1300 stores a boot program such as a basic input output system (BIOS) executed by the CPU 1100 when the computer 1000 is activated, a program dependent on the hardware of the computer 1000, and the like.

The HDD 1400 is a computer-readable recording medium that non-transiently records a program to be executed by the CPU 1100, data used by such a program, and the like. Specifically, the HDD 1400 is a recording medium that records a program according to the present disclosure, which is an example of program data 1450.

The communication interface 1500 is an interface for the computer 1000 to be connected with an external network 1550 (for example, the Internet). For example, the CPU 1100 receives data from another device or transmits data generated by the CPU 1100 to another device via the communication interface 1500.

The input and output interface 1600 is an interface for connecting an input and output device 1650 and the computer 1000. For example, the CPU 1100 receives data from an input device such as a keyboard or a mouse via the input and output interface 1600. The CPU 1100 also transmits data to an output device such as a display, a speaker, or a printer via the input and output interface 1600. Furthermore, the input and output interface 1600 may function as a media interface that reads a program or the like recorded in a predetermined recording medium. A medium refers to, for example, an optical recording medium such as a digital versatile disc (DVD) or a phase change rewritable disk (PD), a magneto-optical recording medium such as a magneto-optical disk (MO), a tape medium, a magnetic recording medium, or a semiconductor memory.

For example, in a case where the computer 1000 functions as the information processing device 100 according to the embodiment, the CPU 1100 of the computer 1000 implements the function of the control unit 140 by executing a program loaded on the RAM 1200. The HDD 1400 also stores a program according to the present disclosure or various types of data. Note that although the CPU 1100 reads the program data 1450 from the HDD 1400 and executes the program data 1450, as another example, these programs may be acquired from another device via the external network 1550.

Incidentally, the effects described in the present specification are merely illustrative or exemplary and are not limiting. That is, the technology according to the present disclosure can achieve other effects that are obvious to those skilled in the art from the description of the present specification together with or in place of the above effects.

Note that the present technology can also have the following configurations.
(1) An information processing method comprising the steps of:
   by a computer,
   estimating stress information regarding a type of stress of a user on a basis of biological information of the user measured in real time by a biological sensor; and
   specifying correlation information correlated with the stress information from behavior information related to a behavior history of the user and activity information related to a social activity history of the user depending on the stress information.
(2) The information processing method according to (1),
   wherein the stress information is estimated on a basis of whether or not a measurement value regarding the biological information indicates an abnormal value.
(3) The information processing method according to (2),
   wherein, in a case where measurement values related to a plurality of types of the biological information indicate abnormal values, the stress information is estimated on a basis of a combination pattern of the abnormal values indicated by the measurement values related to the plurality of types of the biological information.
(4) The information processing method according to any one of (1) to (3),
   wherein the stress information is estimated on a basis of a transition pattern of a measurement value regarding the biological information.
(5) The information processing method according to any one of (1) to (4),
   wherein the biological information is an amount of a hormone in blood of the user.
(6) The information processing method according to any one of (1) to (5),
   wherein the biological information is a blood pressure, a heart rate, a blood glucose level, or electroencephalography of the user.
(7) The information processing method according to any one of (1) to (6),
   wherein the behavior information is position information regarding a position history of the user, search information regarding a search history, browsing information regarding a browsing history, purchase information regarding a purchase history, travel information regarding a travel history, posting information regarding a posting history, or information regarding a history of image data or audio data stored in a terminal device of the user.
(8) The information processing method according to any one of (1) to (7),
   wherein the activity information is personal information managed by a country, a national institution of the country, or a public organization of the country to which the user has ever belonged or an organization to which the user has ever belonged or health information regarding health of the user.
(9) The information processing method according to any one of (1) to (8), further comprising the steps of:
   executing therapy for the user on a basis of the correlation information; and
   outputting question information regarding a question to the user, acquiring reaction information regarding a reaction of the user to the question, and specifying a cause of stress of the user on a basis of the reaction information and the correlation information.
(10) The information processing method according to (9), further comprising the step of:
   specifying a schema of the user on a basis of the reaction information and the correlation information.
(11) The information processing method according to (9) or (10),
   wherein the reaction information is answer information regarding an answer of the user to the question.
(12) The information processing method according to any one of (9) to (11),
   wherein the reaction information is biological information of the user measured during the therapy for the user.
(13) The information processing method according to any one of (9) to (12), further comprising the step of:
   executing a recovery program corresponding to the cause of the stress.
(14) The information processing method according to (13),
   wherein, in a case where a schema of the user is specified, a recovery program for correcting cognitive distortion corresponding to the schema of the user is executed.
(15) An information processing device, comprising:
   an estimation unit that estimates stress information regarding a type of stress of a user on a basis of biological information of the user measured in real time by a biological sensor; and
   a specifying unit that specifies correlation information correlated with the stress information from behavior information related to a behavior history of the user and activity information related to a social activity history of the user depending on the stress information.(16)

An information processing program for causing a computer to function as:
an estimation unit that estimates stress information regarding a type of stress of a user on a basis of biological information of the user measured in real time by a biological sensor; and
a specifying unit that specifies correlation information correlated with the stress information from behavior information related to a behavior history of the user and activity information related to a social activity history of the user depending on the stress information.

### Reference Signs List

- 1: INFORMATION PROCESSING SYSTEM
- 10: SENSOR DEVICE
- 20: INFORMATION PROVIDING DEVICE
- 100: INFORMATION PROCESSING DEVICE
- 110: COMMUNICATION UNIT
- 120: STORAGE UNIT
- 130: OUTPUT UNIT
- 140: INPUT UNIT
- 150: CONTROL UNIT
- 151: ESTIMATION UNIT
- 152: SPECIFYING UNIT
- 153: THERAPY EXECUTION UNIT
- 154: RECOVERY PROGRAM EXECUTING UNIT

## Claims

1. An information processing method comprising the steps of:
by a computer,
estimating stress information regarding a type of stress of a user on a basis of biological information of the user measured in real time by a biological sensor; and
specifying correlation information correlated with the stress information from behavior information related to a behavior history of the user and activity information related to a social activity history of the user depending on the stress information.

2. The information processing method according to claim 1,
wherein the stress information is estimated on a basis of whether or not a measurement value regarding the biological information indicates an abnormal value.

3. The information processing method according to claim 2,
wherein, in a case where measurement values related to a plurality of types of the biological information indicate abnormal values, the stress information is estimated on a basis of a combination pattern of the abnormal values indicated by the measurement values related to the plurality of types of the biological information.

4. The information processing method according to claim 1,
wherein the stress information is estimated on a basis of a transition pattern of a measurement value regarding the biological information.

5. The information processing method according to claim 1,
wherein the biological information is an amount of a hormone in blood of the user.

6. The information processing method according to claim 1,
wherein the biological information is a blood pressure, a heart rate, a blood glucose level, or electroencephalography of the user.

7. The information processing method according to claim 1,
wherein the behavior information is position information regarding a position history of the user, search information regarding a search history, browsing information regarding a browsing history, purchase information regarding a purchase history, travel information regarding a travel history, posting information regarding a posting history, or information regarding a history of image data or audio data stored in a terminal device of the user.

8. The information processing method according to claim 1,
wherein the activity information is personal information managed by a country, a national institution of the country, or a public organization of the country to which the user has ever belonged or an organization to which the user has ever belonged or health information regarding health of the user.

9. The information processing method according to claim 1, further comprising the steps of:
executing therapy for the user on a basis of the correlation information; and
outputting question information regarding a question to the user, acquiring reaction information regarding a reaction of the user to the question, and specifying a cause of stress of the user on a basis of the reaction information and the correlation information.

10. The information processing method according to claim 9, further comprising the step of:
specifying a schema of the user on a basis of the reaction information and the correlation information.

11. The information processing method according to claim 9,
wherein the reaction information is answer information regarding an answer of the user to the question.

12. The information processing method according to claim 9,
wherein the reaction information is biological information of the user measured during the therapy for the user.

13. The information processing method according to claim 9, further comprising the step of:
executing a recovery program corresponding to the cause of the stress.

14. The information processing method according to claim 13,
wherein, in a case where a schema of the user is specified, a recovery program for correcting cognitive distortion corresponding to the schema of the user is executed.

15. An information processing device, comprising:
an estimation unit that estimates stress information regarding a type of stress of a user on a basis of biological information of the user measured in real time by a biological sensor; and
a specifying unit that specifies correlation information correlated with the stress information from behavior information related to a behavior history of the user and activity information related to a social activity history of the user depending on the stress information.

16. An information processing program for causing a computer to function as:
an estimation unit that estimates stress information regarding a type of stress of a user on a basis of biological information of the user measured in real time by a biological sensor; and
a specifying unit that specifies correlation information correlated with the stress information from behavior information related to a behavior history of the user and activity information related to a social activity history of the user depending on the stress information.
